# EUROPEAN PATENT APPLICATION

(11) **EP 4 756 425 A1**
(43) Date of publication of application: **10.06.2026**
(21) Application number: 24218246.7
(22) Date of filing: 09.12.2024
(51) Int. Cl.: G01N 33/28

(54) **METHOD FOR CONTROLLING THE IDENTITY OF A CHEMICAL COMPOSITION**

(71) Applicant: Hilti Aktiengesellschaft, 9494 Schaan (LI)
(72) Inventor: Simon, Sebastian, 86807 Buchloe Lindenberg (DE); Schulte, Kristin, 86916 Kaufering (DE); Bürgel, Thomas, 86899 Landsberg (DE)
(74) Representative: Hilti Aktiengesellschaft Corporate Intellectual Property

(57) **Abstract**

Present invention relates to a method for controlling the identity of a chemical composition Y and a method for authentication of a chemical product X. Present invention further relates to a use of a biobased material in a chemical product as a security marker for authentication purposes.

## Description

In the construction industry, as in all sectors, the more prominent the brand is, the higher the likelihood of encountering counterfeits thereof on the market. Counterfeiters have become more and more sophisticated producing product fakes that are difficult to authenticate even for the experts of the original manufacturers. Very often the counterfeited product is of subpar quality and may comprise inferior and/or more harmful ingredients. The damage is substantial when users mistakenly ascribe the poor quality to the original brand and its products. Such damage to the reputation can require great effort and resources to rectify.

Even without the counterfeit issue, a manufacturer of a construction product needs to be able to check their own products for quality, origin and batches. It would also be advantageous for the possibility to check and confirm the date and location of manufacture and/or to check the compositions brought to market by distributors.

At the present time the most common ways to authenticate a construction product would be to use things like batch information on the packaging, or integrated color pigments. Another solution, specific to chemical anchors, has been disclosed and uses a plurality of different radically curable reactive diluents to identify its products (EP4049990). Such solutions are not always suitable or desirable for a manufacturer. In case of varying the product's pigment, this would not be often desired in view of its user's perception of the quality, usability and the brand identity. Even slight changes in the hue or tint can be perceived by the consumer and are not desirable. There are also many disadvantages in using pigments as authentication markers, since they can be easily replicated, difficult to authenticate on a construction site, and change color hue over time. Use of the special combination of reactive diluents is not always desirable or applicable for different types of construction products. Such a method might also require a new formulation and testing of the product.

In construction industry, products such as chemical anchors and fire protection products are typically based on crude petroleum based raw materials. Thus, there is also a great need for construction products, such as chemical anchors and fire protection products that are more sustainable having a lower carbon footprint and thus having less impact on the climate change crisis. A standard way to reduce product's carbon footprint is to reduce the material consumption such as by using slimmer designs, honeycomb structures instead of solid materials, etc. This approach is not appropriate for chemical anchors or fire protection products, because it would lead to an inferior performance of the product or because it would require a more complicated application which could lead to improper installations and failure of the product as a result thereof.

Another way to lower a carbon footprint of a construction product, such as chemical anchors and fire protection products, would be to substitute some or most of the petroleum-based ingredients with biobased materials or materials derived therefrom.

"Biobased" means containing organic carbon of renewable origin like agricultural, plant, animal, fungi, microorganisms, marine, or forestry materials living in a natural environment in equilibrium with the atmosphere, according to the ASTM D6866-24 analytical standard.

Thus, there is a need for better ways to protect a construction product from counterfeits and at the same time reduce the carbon footprint thereof. There is also a need for a method to authenticate a construction product, wherein the authentic construction product may have the same quality, quality variation and appearance compared to a counterfeit product.

Therefore, the object of the present invention is to provide a method for controlling the identity of a chemical composition, preferably a chemical anchor or a fire protection product. A further objective is to provide a method for authentication of a chemical composition, preferably a chemical anchor or a fire protection product.

Present invention relates to a method for controlling the identity of a chemical composition Y comprising the steps of:
(a) replacing some or all of the at least one component of the chemical composition Y with at least one security marker, which results in a chemical composition Y',
(b) determining the C14 to C12 ratio of the chemical composition Y',
wherein the C14 to C12 ratio of the chemical composition Y' is different from the C14 to C12 ratio of the chemical composition Y, wherein the chemical composition Y' is the authentic product having a defined C14 to C12 ratio, and wherein the security marker is a biobased material derived in whole or in part from biomass resources.

It was surprisingly found that by using the method for controlling the identity of the chemical composition Y according to the present invention, provides a very secure way to mark a chemical product with a security marker, the chemical identity and integrity of which (chemical composition Y') does not change over time differently from the chemical composition Y. Other advantages include that the security marker cannot be detected with traditional methods, such as eye inspection, standard quality control methods or typical analytical means. This allows for the secure marking and identification of the proprietor's products (chemical compositions) not only in-house but also provides the ability to test and verify products' origin in the open market.

It is preferred that the chemical composition Y and the chemical composition Y' have the same physical properties. It is preferred that the chemical composition Y and the chemical composition Y' cannot be differentiated through performance testing typical for the corresponding chemical product or composition. It is preferred that the chemical composition Y and the chemical composition Y' have the same chemical composition, or essentially the same composition, with the only difference being C14 to C12 ratio.

Currently on the market there are many chemicals and chemical products that are in whole or in part derived from biomass resources. Many such products are chemically identical to their crude oil derived chemical/chemical product counterparts having the only difference of C14 to C12 ratio. Thus, it is preferred that the security marker is chemically identical to the components being replaced of the chemical composition Y in (a), wherein the only difference between the security marker and the components being replaced of the chemical composition Y in (a) is the C14 to C12 ratio. In another embodiment there is one, two or more differences between the security marker and the components being replaced in (a). Such differences might include different amounts of impurities, preservatives, additives, stabilizers or mixtures of two or more thereof.

It is preferred that the components that are replaced by at least one security marker in (a) are chemically identical, optionally comprising different amounts of impurities, preservatives, additives, stabilizers, or a mixture of two or more thereof, and the only difference being C14 to C12 ratio between the two. Thus, the security marker may be in whole or in part derived from biomass resources and thus having a different C14 to C12 ratio from the crude oil derived counterparts which correspond to the components being replaced in (a).

Thus, it is preferred that the at least one component of the chemical composition Y, that is being replaced by the security marker in (a) according to the present invention, is derived in part or in whole from crude oil.

It is preferred that the amount of the at least one component being replaced by the security marker in (a) is in the range of from 0.1 to 99.9 weight %, more preferably in the range of from 5 to 90 weight %, more preferably in the range of from 10 to 80 weight %, more preferably in the range of from 25 to 65 weight %, more preferably in the range of from 30 to 70 weight %, more preferably in the range of from 35 to 55 weight % based on the total weight of the at least one component in the chemical composition Y. It is preferred that at least one, more preferably all, more preferably 2 to 20, more preferably 3 to 10, more preferably 4 to 7 of the components in Y are being replace by the security marker in (a).

It is preferred that the chemical composition Y is a construction product, preferably a chemical anchor, any part of the housing for the chemical anchor, a fire protection product or any part of the housing for the fire protection product.

A chemical anchor could in principle be any chemical anchor that comprises components that are derived in part or in whole from crude oil and can be replaced with a corresponding biomass derived counterpart, i.e. a security marker according to the present invention. Thus, it is preferred that at least one component of a chemical anchor would be replaced with at least one security marker in (a) as presently described.

It is preferred that housing for the chemical anchor include but are not limited to plastic packaging, pails, cartridges, tubes, bags and foils.

In case of the chemical anchors and/or housing for the chemical anchors, it is preferred that components that are replaced with a security marker according to the present invention include but are not limited to housing components (plastic packaging, pails, cartridges, tubes, bags, foils), reactive resin components (such as epoxy resins, vinyl resins, polyester resins, epoxy acrylate resins, hybrid resins), raw materials making up the reactive resin components, (such as acrylates, methacrylates and dimethacrylates) reactive diluents, solvents, additives, stabilizers, catalysts, initiators, retardants and combinations of two or more thereof. There are many products on the marked that are made from raw material with bio-carbon content and that derived in whole or in part from biomass resources. Different methacrylates and derivatives thereof are some of the commercially available biobased raw materials. Some examples include, but are not limited to are isosorbide dimethacrylate, PEG200 dimethacrylate (PEG200DMA), isobornyl methacrylate, isopropylendiglycerin dimethacrylate (IPG methacrylate), tetrahydrofurfuryl methacrylate, glycerolformal methacrylate, and isosorbide dimethacrylate (ISDMA).

A fire protection product could in principle be any fire protection product that comprises components that are derived in part or in whole from crude oil and can be replaced with a corresponding biomass derived counterpart, i.e. a security marker according to the present invention. Thus, it is preferred that at least one component of a chemical anchor would be replaced with at least one security marker in (a) as presently described.

It is preferred that the fire protection product is selected from the group comprising firestop sealants, firestop mortars, firestop foams, smoke and/or acoustic sealants, firestop sprays, smoke and/or acoustic sprays, silicone coatings or sprays, steel protection coatings, cable coatings, and intumescent foams.

Housing for the fire protection products include but are not limited to plastic packaging, pails, cartridges, capsules, tubes, bags, and foils.

In case of the fire protection product and/or housing for the fire protection product, it is preferred that components that are replaced with a security marker according to the present invention include but are not limited to plastic components, foil components, reactive resin components, raw materials making up the reactive resin components, reactive diluents, solvents, additives, stabilizers, catalysts, initiators, retardants and combinations of two or more thereof. Some examples of the components that can be replace with a security marker according to the present invention include acrylic dispersions, freeze thaw agents like propylene glycol, butadiene materials like rubbers or pure polybutene, carbon donor like pentaerytrithole and combination of two or more thereof. A person skilled in the art is only limited by the commercial availability of biobased raw materials, ingredients, and reagents, which can replace and are equivalent to their crude oil/fossil oil-derived counterparts.

Present invention relates to a method for authentication of a chemical product X, preferably a chemical anchor or a fire protection product, comprising the steps of:
(i) identifying the C14 to C12 ratio of the chemical product X;
(ii) comparing the C14 to C12 ratio of chemical product X to the C14 to C12 ratio of the chemical product Y',
(iii) establishing if the chemical product X is authentic, wherein the chemical product Y' is the authentic product comprising a security marker, wherein the chemical product Y' has a defined C14 to C12 ratio, and wherein the security marker is a biobased material derived in whole or in part from biomass resources.

It is preferred that the chemical product X is authenticated (or establishing if the chemical product X is authentic) if the C14 to C12 ratio is within 0.01 to 5 % error, more preferably is within 0.1 to 2 % error, more preferably is within 0.5 to 1 % error, compared to the chemical product Y'.

The method according to the present invention can also be used to not only authenticate products but also to confirm production quality, source of batch materials, and any other information that needs to be tranced for quality control in the industrial setting.

As disclosed herewith a chemical anchor or a fire protection product, can be any product that can be tested for C14 to C12 ratio.

It is preferred that a chemical anchor is a multi-component reactive system. In principle it can be any chemical anchor comprising a crude oil-based component that can be replaced by its biobased material counterpart derived in whole or in part from biomass resources. It is preferred that the chemical anchors are epoxy based, polyurea based, vinylester based, polyester based, acrylic and/or methacrylic based adhesive anchors. It is preferred that the fire protection product is selected from the group comprising firestop sealants, firestop mortars, firestop foams, smoke and/or acoustic sealants, firestop sprays, smoke and/or acoustic sprays, silicone coatings or sprays, steel protection coatings, cable coatings, and intumescent foams.

As disclosed herewith the housing for the chemical anchor or the housing for the fire protection product can be also authenticated according to the method of the present invention.

In theory any standardized method for measuring C14 to C12 ratio can be used for the method of authenticating according to the present invention.

It is preferred that the identification of the C14 to C12 ratio is carried out according to ISO 13833 and DIN 16640.

Present invention also relates to a use of a biobased material in a chemical product as a security marker for authentication purposes, wherein the chemical product is a chemical anchor or a fire protection product.

### Examples

The examples according to the present invention are not limiting. At the present time, a person skilled in the art is limited only be the commercial availability of bio-based equivalents of the crude oil produced counterpart of reagents, raw materials, and the like. Thus, the following examples are there to demonstrate possible formulations of chemical anchors that would be applicable for the method for controlling the identity of a chemical composition according to the present invention.

**Table 1: Ingredients**

| **Name** | **Description** | **Supplier** | **Fossil carbon** | **Bio-carbon** | **Bio-content in weight %** |
|---|---|---|---|---|---|
| UMA-121 | urethane methacrylate resin according to WO2019/007667, example C1: | -- | | 0 | 0 |
| | the resin was prepared and further used as a master batch in mixing with BDDMA in a mass ratio of 65/35 | | | | |
| E3BADMA | Ethoxylated 3 Bisphenol A DiMethacrylate | Arkema | | 0 | 0 |
| ISDMA | Isosorbide dimethacrylate | Evonik | 8 | 6 | 42.9 |
| PEG200DMA | PEG200 dimethacrylate | Evonik | 8 | 8 | 50 |
| IPGMA | Isopropylendiglycerin dimethacrylate | Evonik | 4 | 6 | 60 |
| THFMA | Tetrahyrofurfuryl methacrylate | Evonik | 4 | 5 | 55.6 |
| GlyFoMA | Glycerolformal methacrylate | Evonik | 4 | 4 | 50 |
| IBOMA | Isobornyl methacrylate | Evonik | 4 | 10 | 71.4 |
| N,N-Bis(hydroxyethyl)-p-toluidine | N,N-Bis(hydroxyethyl)-p-toluidine | | 11 | 0 | 0 |
| p-tert. butyl-pyrocatechol | p-tert. butyl-pyrocatechol | | 10 | 0 | 0 |
| Dibenzoylperoxide | Dibenzoylperoxide | | 14 | 0 | 0.0 |
| Glycerine | Glycerine | | 0 | 3 | 100.0 |

Fossil carbon refers to carbons that are derived from crude oil materials. Bio-carbon refers to carbons derived from biobased materials as presently defined.

**Table 2: Resins 1 to 3**

| **Resin mixtures** | **Composition (organic materials only) wt.-%** | | | **Bio-carbon content in Mol% carbon** | | |
|---|---|---|---|---|---|---|
| | **Resin 1** | **Resin 2** | **Resin 3** | **Resin 1** | **Resin 2** | **Resin 3** |
| UMA-121 | 30 | | 15 | 0.0 | 0.0 | 0.0 |
| E3BADMA | | 60 | | 0.0 | 0.0 | 0.0 |
| ISDMA | | | 43.3 | 0.0 | 0.0 | 18.6 |
| PEG200DMA | 48.3 | | 30 | 24.2 | 0.0 | 15.0 |
| IPGMA | 20 | | | 12.0 | 0.0 | 0.0 |
| THFMA | | | 10 | 0.0 | 0.0 | 5.6 |
| GlyFoMA | | 18.35 | | 0.0 | 9.2 | 0.0 |
| IBOMA | | 20 | | 0.0 | 14.3 | 0.0 |
| Bis(hydroxyethyl)-p-toluidine | 1.5 | 1.5 | 1.5 | 0.0 | 0.0 | 0.0 |
| p-tert. butyl-pyrocatechol | 0.2 | 0.15 | 0.2 | 0.0 | 0.0 | 0.0 |
| Total | 100 | 100 | 100 | 36.2 | 23.5 | 39.2 |

**Table 3: A-Component 1 to 3**

| | **A-comp. 1** | **A-comp. 2** | **A-comp. 3** |
|---|---|---|---|
| Resin 1 | 37.5 | | |
| Resin 2 | | 40 | |
| Resin 3 | | | 45 |
| Fumed silica | 2.5 | 3 | 3.5 |
| Quartz sand | 40 | 37 | 31.5 |
| Quartz flour | 20 | | |
| Alumina cement | | 20 | 20 |
| | | | |
| Total weight % | 100 | 100 | 100 |

**Table 4: B-Components 1 and 2**

| **Ingredients** | **B-comp. 1** | **B-comp. 2** | **Bio-content (B-comp. 1)** | **Bio-content (B-comp. 2)** |
|---|---|---|---|---|
| Dibenzoylperoxide | 15 | 25 | 0 | |
| Water | 35 | | | |
| Glycerine | | 35 | | 58.3 |
| Fumed silica | 2.5 | 1.5 | | |
| Quartz flour | 47.5 | 38.5 | | |
| bio-carbon content in Mol% carbon | 0 | 58.3 | | |
| Total weight % | 100 | 100 | 0 | 58.3 |

**Table 5: Chemical Anchors (Examples 1 to 3)**

| | **Ratio** | | | **Organic content in mixture A+B** | | | | **Bio-content in organic material** | | |
|---|---|---|---|---|---|---|---|---|---|---|
| | **Ex.1** | **Ex.2** | **Ex. 3** | **Ex.1** | **Ex.2** | **Ex.3** | | **Ex.1** | **Ex.2** | **Ex.3** |
| **A-comp. 1** | 5 | | | 31.3 | | | | 33.5 | | |
| **A-comp. 2** | | 5 | | | 33.3 | | | | 21.8 | |
| **A-comp. 3** | | | 10 | | | 40.9 | | | | 34.5 |
| | | | | | | | | | | |
| **B-comp. 1** | 1 | 1 | | 2.5 | 2.5 | | | 0 | 0 | |
| **B-comp. 2** | | | 1 | | | 5.5 | | | | 6.9 |
| | | | | | | | | | | |
| **parts by weight wt.-%** | 6 | 6 | 11 | 33.8 | 35.8 | 46.4 | **bio-carbon content in Mol% carbon** | 33.5 | 21.8 | 41.4 |

As can be seen from the present examples, all or parts of the fossil derived organic content of the ingredients in either A-component or B-component can be replaced with respective biobased counterpart, which according to the present invention serves as a security marker. According to the present invention, such replacement is used to control the identity of the product or used in the authentication of a questionable counterfeit. Further, labeling with the securing marker according to the present invention and such as shown in the examples according to the present invention, can be used to trace batch source/origin and for general quality control of the production process. According to the present invention C14 to C12 ratio testing should be used to authenticate or identify the authentic product or detect a counterfeit. It is preferred that the identification of the C14 to C12 ratio is carried out according to ISO 13833 and DIN 16640.

## Claims

1. A method for controlling the identity of a chemical composition Y comprising the steps of:
(a) replacing some or all of the at least one component of the chemical composition Y with at least one security marker, which results in a chemical composition Y',
(b) determining the C14 to C12 ratio of the chemical composition Y',
wherein the C14 to C12 ratio of the chemical composition Y' is different from the C14 to C12 ratio of the chemical composition Y, wherein the chemical composition Y' is the authentic product having a defined C14 to C12 ratio, and wherein the security marker is a biobased material derived in whole or in part from biomass resources.

2. The method according to claim 1, wherein the at least one component of the chemical composition Y is derived in part or in whole from crude oil.

3. The method according to claim 1 or 2, wherein the chemical composition Y is a chemical anchor or a fire protection product.

4. The method according to claim 3, wherein the chemical anchor is a multi-component reactive system.

5. The method according to claim 3, wherein the fire protection product is selected from the group comprising firestop sealants, firestop mortars, firestop foams, smoke and/or acoustic sealants, firestop sprays, smoke and/or acoustic sprays, silicone coatings or sprays, steel protection coatings, cable coatings, and intumescent foams.

6. A method for authentication of a chemical product X, preferably a chemical anchor or a fire protection product, comprising the steps of:
(i) identifying the C14 to C12 ratio of the chemical product X;
(ii) comparing the C14 to C12 ratio of chemical product X to the C14 to C12 ratio of the chemical product Y',
(iii) establishing if the chemical product X is authentic,
wherein the chemical product Y' is the authentic product comprising a security marker, wherein the chemical product Y' has a defined C14 to C12 ratio, and wherein the security marker is a biobased material derived in whole or in part from biomass resources.

7. The method for authentication according to claim 6, wherein the chemical product X is authenticated if the C14 to C12 ratio is within 0.01 to 5 % error, more preferably is within 0.1 to 2 % error, more preferably 0.5 to 1 % error compared to the chemical product Y'.

8. The method for authentication according to claim 6 or 7, wherein the chemical product X is a chemical anchor, preferably wherein the chemical anchor is a multi-component reactive system.

9. The method for authentication according to claim 6 or 7, wherein the chemical product X is a fire protection product, preferably the fire protection product is selected from the group comprising firestop sealants, firestop mortars, firestop foams, smoke and/or acoustic sealants, firestop sprays, smoke and/or acoustic sprays, silicone coatings or sprays, steel protection coatings, cable coatings, and intumescent foams.

10. The method for authentication according to any one of claims 6 to 9, wherein the identification of the C14 to C12 ratio is carried out according to ISO 13833 and DIN 16640.

11. A use of a biobased material in a chemical product as a security marker for authentication purposes, wherein the chemical product is a chemical anchor or a fire protection product.
